# EUROPEAN PATENT SPECIFICATION

(11) **EP 2 111 241 B1**
(45) Date of publication and mention of the grant of the patent: **05.01.2011**
(21) Application number: 07867470.2
(22) Date of filing: 15.11.2007
(51) Int. Cl.: A61L 31/10, A61L 31/16

(54) **STENT WITH DIFFERENTIAL TIMING OF ABLUMINAL AND LUMINAL RELEASE OF A THERAPEUTIC AGENT**
STENT MIT FUNKTION ZUR ZU UNTERSCHIEDLICHEN ZEITEN ERFOLGENDEN ABLUMINALEN UND LUMINALEN FREISETZUNG EINES BEHANDLUNGSMITTELS
ENDOPROTHÈSE VASCULAIRE AVEC CADENCEMENT DIFFÉRENTIEL DE LIBÉRATION ABLUMINALE ET LUMINALE D'UN AGENT THÉRAPEUTIQUE

(30) Priority: 16.11.2006 US 859977 P
(43) Date of publication of application: 28.10.2009
(73) Proprietor: Boston Scientific Limited, Christ Church (BB)
(72) Inventor: SHIPPY, James Lee, Wilmington, 28411 NC (US); ROBERTSON, Kimberly A., Forest Lake, MN 55025 (US); WEBER, Jan, 6228 GJ Maastricht (NL)
(74) Representative: Hauck Patent- und Rechtsanwälte
(86) International application number: PCT/US2007/024012
(87) International publication number: WO 2008/063539

(56) References cited:
- WO-A-2004/043509
- WO-A-2006/065685
- US-A1- 2002 165 608
- US-A1- 2004 059 409
- US-A1- 2006 093 643
- US-B1- 6 660 034

## Description

### Field Of The Invention

The invention relates generally to a medical device that is useful for delivering a therapeutic agent to the body tissue of a patient, and the method for making such a medical device. More particularly, the invention is directed to a stent having a tubular sidewall with a first coating composition on at least the luminal surface and/or abluminal surface, and a second coating composition disposed on at least a portion of the first coating composition comprising a biodegradable polymer.

### Background Of The Invention

Angioplasty is a procedure that involves placing and inflating a balloon catheter in the blood vessel in the area of blockage, which breaks up the accumulated plaque and opens the vessel. While this technique works well in the short term, many angioplasty procedures require further treatment within six months because of incomplete plaque removal and formation of scar tissue as a result of irritation of the blood vessel, known as restenosis. Restenosis results in significant morbidity and mortality and requires further interventions such as repeat angioplasty, coronary bypass, laser surgery or local drug delivery.

Intravascular stenting (the placement of a supporting structure within a blood vessel) has demonstrated success in preventing further interventions in patients. Stents provide structural support to keep the vessel walls from closing and minimize the problem of artertial blockage caused by plaque falling into the vessel after inflation. Intravascular stenting is now commonly involved in 70-90% of procedures performed. Stents can be used as an alternative to or in combination with angioplastly.

Exposure to stents, which are implanted or inserted into the body of a patient, can cause the body tissue to exhibit adverse physiological reactions. For instance, the insertion or implantation of certain stents can lead to the formation of emboli or clots in blood vessels. Other adverse reactions to stents include cell proliferation which can lead to hyperplasia, occlusion of blood vessels, platelet aggregation, and calcification.

In order to address such adverse effects, stents have incorporated therapeutic agents. Such materials can be incorporated into the materials used to make the device. Alternatively, the therapeutic agent(s) can be included in a coating that is applied to a surface of the medical device.

Stents that include a therapeutic agent can be used for direct or local administration of the therapeutic agent to a particular part of the patient's body. For instance, stents having coatings that include a therapeutic agent can be used to prevent restenosis. In some instances, the coating can also include a polymeric material that controls the delivery or release of the therapeutic agent. For example, various types of coated stents in which the coating includes a therapeutic agent have been used for localized delivery of drugs to a body lumen. *See, e.g*., U.S. Patent No. 6,099,562 to Ding et al.

Such direct or local administration may be more preferred than systemic administration of a therapeutic agent. Systemic administration requires larger amounts and/or higher concentrations of the therapeutic agent because of indirect delivery of such materials to the afflicted area. Also, systemic administration may cause side effects which may not be a problem when the therapeutic agent is locally administered.

Although there are many advantages to incorporating a therapeutic agent into a stent, computational fluid dynamics models for implanted stents coated with a therapeutic agent show that about 1/5 of the total amount of therapeutic agent that is released from the stent is delivered to the blood vessel wall, while about 4/5 of the total amount of therapeutic agent released from the stent is delivered to the blood stream. In particular, these models indicate that a burst release of the therapeutic agent into the bloodstream occurs within 48 hours of implantation or delivery of the stent. In general, the therapeutic agent located on the luminal surface of the stent or stent struts, or located on the side surfaces of the stent struts, is released into the bloodstream during this burst release. This burst release is followed by a lower sustained release of the therapeutic agent.

After implantation of the stent, it is intended that, the stent or struts of the stent should become covered with a new intima layer consisting of endothelial cells. This cell growth occurs generally around the parts of the stent that are exposed to the bloodstream. To minimize excessive neo-intima thickening, which may result from arterial and vascular injuries causing thrombus and inflammatory responses, it might be desirable to deliver therapeutic agents to the intima layer to suppress hyperplasia. However, because a large percentage of the burst release of the therapeutic agent is lost to the bloodstream, less therapeutic agent remains to be released to the neointimal layer during the sustained release period.

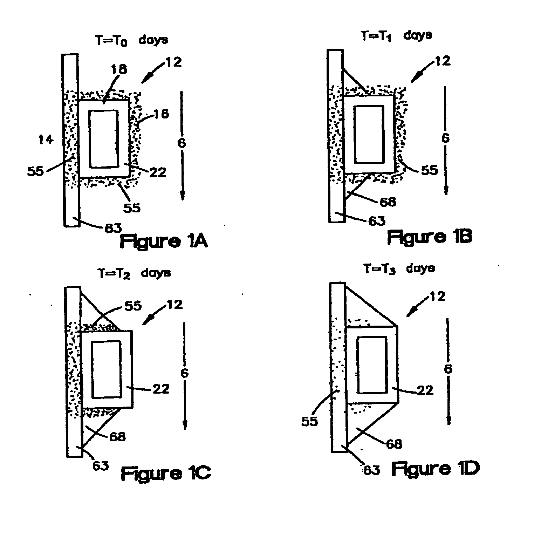

**Figures 1a-1d** depict how the burst release of the therapeutic agent causes it to be lost to the bloodstream so that only a limited amount of the therapeutic agent remains to be released to the intima growth. As shown in **Figure 1a**, when a stent having a strut **12** is implanted in a blood vessel **63,** the therapeutic agent **55** is immediately released from the coating composition **22** applied to the side surfaces 18 and luminal surface **16** of a stent strut **12** to the bloodstream 6. **Figures 1b-1d****,** show that as the neointimal tissue made of endothelial cells **68** grows around the strut, a greater percentage of the therapeutic agent **55** released from the strut at a given time is absorbed into the tissue **68.** However by the time this tissue **68** forms, the release of the therapeutic agent **55** has slowed down dramatically. As a result, the majority of the therapeutic agent **55** released during the first few weeks does not act on the tissue **68.** This results in a low concentration of the therapeutic agent **55** left for delivery to target neo-intima thickening from the endothelial growth, as shown in **Figures 1b-1d****.**

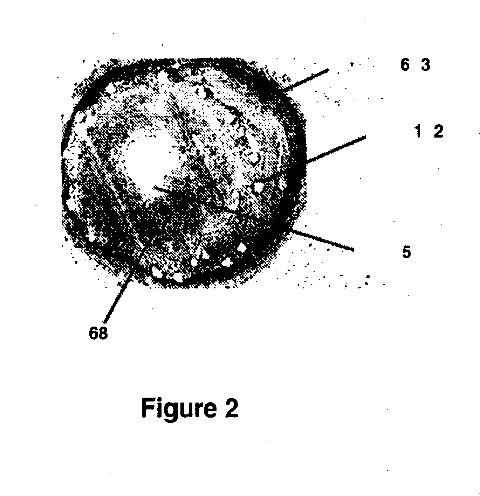

When directly adjacent tissue is not exposed to therapeutic agent or not exposed to sufficient therapeutic agent, occlusion and restenosis can result despite the loading of therapeutic agent onto or into the stent. **Figure 2** shows a cross section of a stent **10** having struts **12** implanted in a blood vessel **63.** Within days after implantation, the endothelial cells **68** begin to encapsulate the stent struts. However platelets, fibrin and neutrophils accumulate as well at the stent site. At 14 to 30 days, chronic inflammation may develop and smooth muscle cells can begin to protrude from abluminal to luminal stent areas, causing a hyperplasia effect whereby the intimal layer **5** thickens to the point of occlusion and restenosis as shown in **Figure 2****.** Without effective delivery of a therapeutic agent to target undesired cell growth, such cell growth continues to occur radially inward towards the center of the blood vessel.

Also, in some instances it is desirable to deliver a therapeutic agent to certain portions of a body lumen by selectively coating or placing the therapeutic agent on certain parts of the stent or stent struts. For example, it may be desirable to only coat the abluminal surface of a stent strut with a therapeutic agent because the preferred endothelial coverage on the luminal site can be reduced by the same therapeutic substance that is targeted to suppress smooth muscle cell growth. However, it may be difficult to only coat one surface of a stent strut with the therapeutic agent because of limitations in the adhesion of the coating containing the therapeutic agent to the strut surface.

Document WO 2006/065685 A2 shows coatings for implantable medical devices such as stents. The devices may include at least one structural element having an abluminal side, a luminal side and side walls between the abluminal and the luminal sides. The coating may include at least two continuous coating layers. In some embodiments, the luminal side and all or a majority of the side walls are free of at least two of the coating layers.

US publication No. US 2004/0059409 A1 shows a stent having a tubular side wall, wherein the side wall is comprised of a plurality of struts each having an outer surface, an inner surface opposite the outer surface, and at least one side surface adjacent to and connecting the outer and inner surfaces. The outer surface or the inner surface is covered at least in part with a first coating. The outer surface or inner surface not covered with a first coating is covered with a second coating.

From US patent No. 6,660,034 B1 an implantable stent for a vessel has become known including a tubular body having an inner and outer surface. The inner and outer surfaces are coated with therapeutic substances.

From US publication No. US 2006/0093643 A1 medical devices have become known comprising a surface coated by at least two coating regions comprising a therapeutic agent. The coating regions allow for the release of the therapeutic agent over different time periods.

From document WO 2004/043509 A1 an expandable medical device for delivery of angiogenic agents has become known including openings in the expandable medical device struts to deliver one or more angiogenic agents to promote angiogenesis. The device can sequentially deliver a plurality of agents.

From US publication No. US 2002/0165608 A1 medical devices for local drug delivery have become known. These utilise various materials and coating methodologies to maintain the drugs, agents or compounds on the medical device until delivered and positioned.

In sum, ineffective delivery of a therapeutic agent results in loss of the therapeutic agent to the blood stream, and inadequate control of undesired cell growth. Thus, there is a need for a stent having a therapeutic agent that can remain on or in the stent over a desired period of time to address such undesired cell growth, which develops only weeks after implanting the stent. In particular, there is a need for a stent having a therapeutic agent where the therapeutic agent is not released in a burst but is released at a lower rate so that there is an adequate amount of the therapeutic agent for addressing the undesired cell growth. Also, there is a need to selectively coat stent surfaces with a therapeutic agent.

### Summary of the Invention

These and other objectives are accomplished by the present invention. The present invention is directed to a medical device with a therapeutic agent coating designed to prevent loss of therapeutic agent to the blood stream, to retain therapeutic agent for delivery to cells, to expose all cells to desired therapeutic agent conditions, and modulate therapeutic agent release as desired. The present invention is directed to a stent that comprises a means of delaying the release of a therapeutic agent from certain parts of a medical device. The present invention provides a coating composition and/or layers of coating compositions that reduces or delays the release of a therapeutic agent. Upon deployment of the stent in a blood vessel, the therapeutic agent is initially held back wholly or partly by a release-limiting, biodegradable coating composition. Over time as endothelial cells encapsulate the stent struts and the coating composition is absorbed into the surrounding cell growth, the therapeutic agent is allowed to be released from the surface as governed by mass transport mechanisms and kinetic drug release (KDR) profiles.

The invention is an intravascular stent designed for implantation into a blood vessel of a patient. The stent comprises a tubular stent sidewall structure comprising a plurality of struts and openings in the sidewall structure. At least one strut comprises an abluminal surface, and a luminal surface opposite the abluminal surface. There is a first coating composition comprising a first polymer and a therapeutic agent disposed on the abluminal surface and luminal surface. There is a second coating composition disposed on a portion of the first coating composition that is disposed on the the luminal surface. The second coating composition is biodegradable and comprises a bioabsorbable polymer; characterized in that the abluminal surface is free of the second coating composition and when the stent is implanted, the first coating compostion disposed on the abluminal surface is in direct contact with the blood vessel.

In one example, there is disclosed a method of making an intravascular stent designed for implantation into a blood vessel of a patient The method comprises a tubular stent sidewall structure comprising a plurality of struts and openings in the sidewall structure. At least one strut comprises an abluminal surface in direct contact with the vessel wall, and a luminal surface opposite the abluminal surface in contact with the blood stream. The method comprises forming a first coating composition comprising a first polymer and a therapeutic agent and disposing the first coating composition on the abluminal surface and luminal surface. The method also comprises forming a second coating composition wherein the second coating composition is biodegradable and comprises a bioabsorbable polymer and disposing the second coating composition on a portion of the first coating composition that is disposed on the luminal surface.

### Brief Description of the Drawing.

Figures 1a-1d show a stent strut with endothelial cell growth and loss of a therapeutic agent to the bloodstream over time.
Figure 2 is a photograph depicting a perspective view of an implanted intravascular stent where endothelialization of the stent has occurred to the point of occlusion and restenosis.
Figure 3 shows an example of an intravascular stent having a middle portion disposed between two end portions.
Figure 3a shows the abluminal, luminal, and side surface of a strut of Figure 3.
Figure 3b shows a cross-section of a stent with struts including the abluminal, luminal and side surfaces of a strut of Figure 3.
Figures 4a-4d depict a strut of a stent, which is described herein, having a coating composition releasing therapeutic agent over time.

### Detailed Description of the Invention

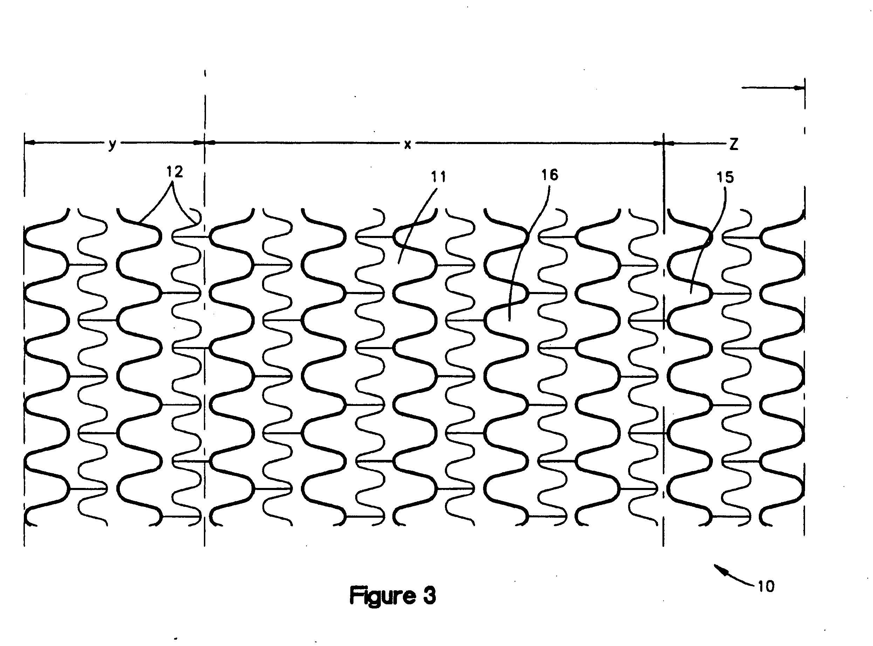

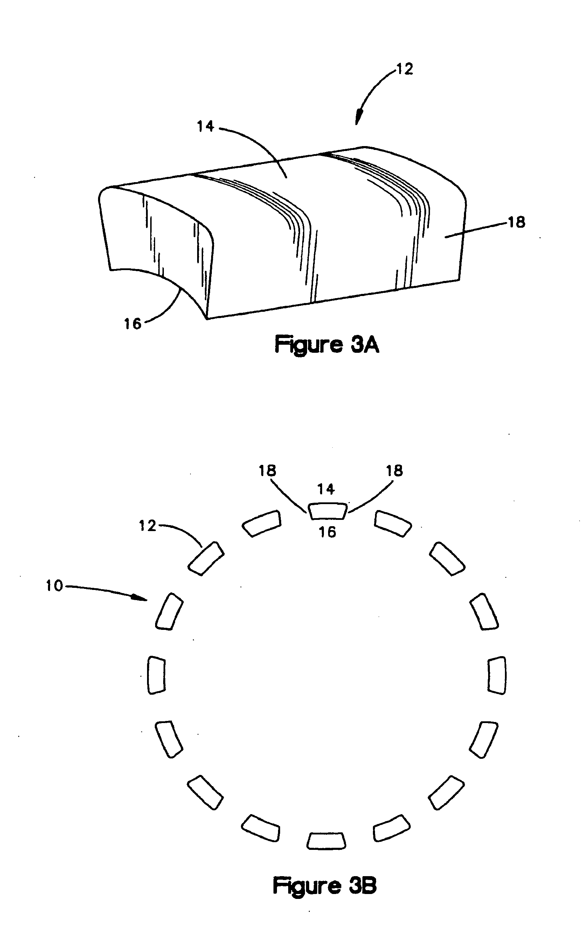

**Figure 3** shows an example of a medical device that is suitable for use in the present invention. This figure shows an implantable intravascular stent **10** comprising a sidewall **11** which comprises a plurality of struts **12** and at least one opening **15** in the sidewall **11.** Generally, the openings **15** are disposed between adjacent struts **12.** This embodiment is an example of a stent where the struts and openings of the stent define a sidewall stent structure having openings therein. Also, the sidewall **11** may have a first sidewall surface **16** and an opposing second sidewall surface, which is not shown in **Figure 3****.** The first sidewall surface **16** can be an outer sidewall surface, which faces the body lumen wall when the stent is implanted, or an inner sidewall surface, which faces away from the body lumen wall. Likewise, the second sidewall surface can be an outer sidewall surface or an inner sidewall surface. The stent **10** comprises a middle portion x and two end portions y and z. Generally, the combined end sections comprise about 20% or less of the overall length of the stent.

**Figure 3a** is a cross-sectional view of a stent strut **12** depicted in **Figure 3****.** Generally, each individual strut **12** has an outer surface or abluminal surface **14,** an inner surface **16** opposite the outer surface, or luminal surface **14,** and at least one side surface **18.** **Figure 3b** depicts a cross section of a stent **10** with struts. The abluminal surface **14** of the strut **12** is the surface that comes in direct contact with the body lumen wall when the stent is implanted. The abluminal surface **14** need not include only one flat surface or facet. Instead, it can be rounded, such as in the case of a wire strut **12,** or have a number of facets. The luminal surface **16** of the strut **12** is the surface that is opposite the abluminal surface **14.** The two side surfaces **18** are the surfaces of the strut **12** that are adjacent to the luminal surface **16** and abluminal surface **14.** The side surface **18** connects the luminal surface **16** and the abluminal surface **14.** Like the abluminal surface **14,** the luminal surface **16** and side surfaces **18** can be rounded or have a number of facets.

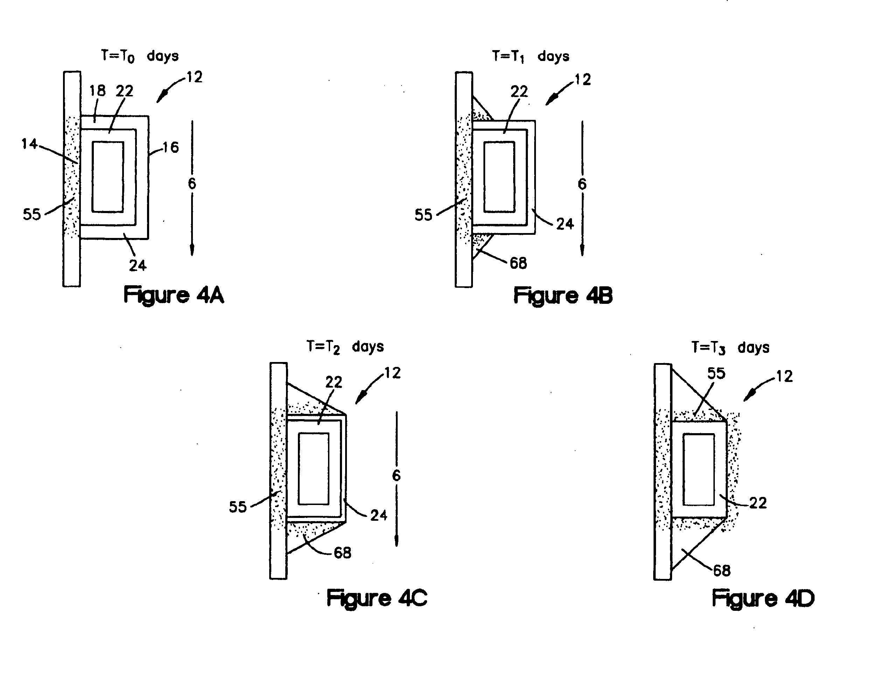

**Figures 4a-4d** depicts one embodiment of the present invention. In this embodiment, a first coating composition **22** is disposed on the abluminal surface **14,** luminal surface **16,** and side surfaces **18** of a stent strut **12.** There is a second coating composition **24** disposed on the portion of the first coating composition **22** that is disposed on the luminal surface **16** and side surfaces **18.** Unlike in **Figures 1a-1d****,** the second coating composition **24** prevents the therapeutic agent **55** from being released too quickly into the bloodstream. As **Figures 4b** and **4c** show, over time endothelial cell growth **68** occurs and the second coating composition **24** degrades. In **Figure 4d****,** when the second coating composition **24** has degraded, the therapeutic agent **55** can be released to the endothelial cells **68** which surround the stent strut **12.** As a result, there is sufficient therapeutic agent **55** to treat the endothelial cells **68.**

In one embodiment, the first coating composition **22** comprises a first polymer and a therapeutic agent **55.** The first polymer is preferably biostable. In other aspects of the invention, the first coating composition **22** can comprise one or more therapeutic agents. It is also possible that more than one polymer is used to form the first coating composition **22.** The second coating composition **24** comprises a biodegradable or bioabsorbable polymer. It is possible that the second coating composition **24** also contains therapeutic agent **55** or is free of any therapeutic agent when applied to the first coating composition. The polymer of the first coating composition **22** and the second coating composition **24** can be the same or different. In the event both polymers of the first coating composition **22** and the second coating composition **24** are biodegradable or bioresorbable, the first coating composition **22** must degrade at a slower rate than the second coating composition **24.** The therapeutic agents in the first and the second coating composition can be the same or different.

In alternative embodiments not shown, it is possible that first coating composition **22** is only disposed on the abluminal surface 14, and luminal surfaces **16.** It is also possible that the first coating composition **22** is disposed on the abluminal surface **14,** and the side surfaces **18,** or the luminal surfaces **16,** and the side surfaces **18.** The second coating composition can be disposed on at least the portion of the first coating composition **22** that is disposed on the luminal surfaces **16** and side surfaces **18;** or only the luminal surface **16.**

### A. Medical Devices

Medical devices that are particularly suitable for the present invention include any kind of stent for medical purposes which is known to the skilled artisan. Preferably, the stents are intravascular stents that are designed for permanent implantation in a blood vessel of a patient and that have an sidewall stent structure with openings. Suitable intravascular stents include self expanding stents and balloon expandable stents. Examples of self expanding stents useful in the present invention are illustrated in U.S. Patent Nos. 4,655,771 and 4,954,126 issued to Wallsten and 5,061,275 issued to Wallsten et aL Examples of appropriate balloon expandable stents are shown in U.S. Patent No. 5,449,373 issued to Pinchasik et al. In certain embodiments, the stent comprises a sidewall stent structure with openings. When such stents are used, it is in some instances preferable to have the coating disposed on the stent to conform to the stent to preserve the openings of the sidewall structure. In preferred embodiments, the stent suitable for the present invention is an Express stent More preferably, the Express stent is an Express™ stent or an Express2™ stent (Boston Scientific, Inc. Natick, Mass.).

Stents that are suitable for the present invention may be fabricated from metallic, ceramic, polymers, or a combination thereof. Preferably, the materials are biocompatible. Metallic material is more preferable. Suitable metallic materials include metals and alloys based on titanium (such as nitinol, nickel titanium alloys, thermo memory alloy materials), stainless steel, niobium, tantalum, nickel chrome, or certain cobalt alloys including cobalt chromium nickel alloys such as Elgiloy® and Phynox®. Metallic materials also include clad composite filaments, such as those disclosed in WO 94/16646.

Suitable ceramic materials include, but are not limited to, oxides, carbides, or nitrides of the transition elements such as titanium oxides, hafnium oxides, iridiumoxides, chromium oxides, aluminum oxides, and zirconiumoxides. Silicon based materials, such as silica, may also be used. The polymer may be biostable. Also, the polymer may be biodegradable. Suitable polymers include, but are not limited to, styrene isobutylene styrene, polyetheroxides, polyvinyl alcohol, polyglycolic acid, polylactic acid, polyamides, poly-2-hydroxy-butyrate, polycaprolactone, poly(lactic-co-clycolic)acid, and Teflon.

Polymers may be used for forming the stent in the present invention include without limitation isobutylene-based polymers, polystyrene-based polymers, polyacrylates, and polyacrylate derivatives, vinyl acetate-based polymers and its copolymers, polyurethane and its copolymers, silicone and its copolymers, ethylene vinyl-acetate, polyethylene terephtalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polylactic acid, polyglycolic acid, polycaprolactone, polylactic acid-polyethylene oxide copolymers, cellulose, collagens, and chitins.

Other polymers that are useful as materials for stents include without limitation dacron polyester, poly(ethylene terephthalate), polycarbonate, polymethylmethacrylate, polypropylene, polyalkylene oxalates, polyvinylchloride, polyurethanes, polysiloxanes, nylons, poly(dimethyl siloxane), polycyanoacrylates, polyphosphazenes, poly(amino acids), ethylene glycol I dimethacrylate, poly(methyl methacrylate), poly(2-hydroxyethyl methacrylate), polytetrafluoroethylene poly(HEMA), polyhydroxyalkanoates, polytetrafluorethylene, polycarbonate, poly(glycolide-lactide) copolymer, polylactic acid, poly(γ-caprolactone), poly(γ -hydroxybutyrate), polydioxanone, poly(γ -ethyl glutamate), polyiminocarbonates, poly(ortho ester), polyanhydrides, alginate, dextran, chitin, cotton, polyglycolic acid, polyurethane, or derivatized versions thereof, i.e., polymers which have been modified to include, for example, attachment sites or cross-linking groups, *e.g*., RGD, in which the polymers retain their structural integrity while allowing for attachment of cells and molecules, such as proteins, nucleic acids, and the like.

Stents may also be made with non-polymers. Examples of useful non-polymers include sterols such as cholesterol, stigmaterol, β-sitosterol, and estradiol; cholesteryl esters such as cholesteryl stearate; C₁₂ -C₂₄ fatty acids such as lauric acid, myristic acid, palmitic acid, stearic acid, arachidic acid, behenic acid, and lignoceric acid; C₁₈ -C₃₆ mono-, di- and triacylglycerides such as glyceryl monooleate, glyceryl monolinoleate, glyceryl monolaurate, glyceryl monodocosanoate, glyceryl monomyristate, glyceryl monodicenoate, glyceryl dipalmitate, glyceryl didocosanoate, glyceryl dimyristate, glyceryl didecenoate, glyceryl tridocosanoate, glyceryl trimyristate, glyceryl tridecenoate, glycerol tristearate and mixtures thereof; sucrose fatty acid esters such as sucrose distearate and sucrose palmitate; sorbitan fatty acid esters such as sorbitan monostearate, sorbitan palpitate and sorbitan tristearate; C₁₆ -C₁₈ fatty alcohols such as cetyl alcohol, myristyl alcohol, stearyl alcohol, and cetostearyl alcohol; esters of fatty alcohols and fatty acids such as cetyl palmitate and cetearyl palmitate; anhydrides of fatty acids such as stearic anhydride; phospholipids including phosphatidylcholine (lecithin), phosphatidylserine, phosphaddylethanolamine, phosphatidylinositol, and lysoderivatives thereof; sphingosine and derivatives thereof; sphingomyelins such as stearyl, palmitoyl, and tricosanyl sphingomyelins; ceramides such as stearyl and palmitoyl ceramides; glycosphingolipids; lanolin and lanolin alcohols; and combinations and mixtures thereof. Preferred non-polymers include cholesterol, glyceryl monostearate, glycerol tristearate, stearic acid, stearic anhydride, glyceryl monooleate, glyceryl monolinoleate, and acetylated monoglycerides.

The stent of the present invention is made of biodegradable materials that are also biocompatible. Biodegradable means that a material will undergo breakdown or decomposition into harmless compounds as part of a normal biological process. In preferred embodiments, the invention comprises using bioabsorbable polymers. The bioabsorbable polymer can comprise polyelectrolyte components such as heparin, albumin, and gelatin. In one embodiment, the bioabsorbable polymer degrades at a rate of about 95% weight loss per 30 days to about 95% weight loss per hour. The stent may have a plurality of apertures in the body of the to stent promote the successful biodegradation of the stent. Suitable biodegradable materials for the stent include polylactic acid, polyglycolic acid (PGA), collagen or other connective proteins or natural materials, polycaprolactone, hylauric acid, adhesive proteins, copolymers of these materials as well as composites and combinations thereof and combinations of other biodegradable polymers. Biodegradable glass or bioactive glass is also a suitable biodegradable material for use in the present invention. Preferably the materials have been approved by the Food and Drug Adminstration.

### B. Suitable Therapeutic Agents

The term "therapeutic agent" encompasses biologically active material, and also genetic materials and biological materials. The therapeutic agents named herein include their analogs and derivatives. Non-limiting examples of suitable therapeutic agent include heparin, heparin derivatives, urokinase, dextrophenylalanine proline arginine chloromethylketone (PPack), enoxaprin, angiopeptin, hirudin, acetylsalicylic acid, tacrolimus, everolimus, rapamycin (sirolimus), pimecrolimus, amlodipine, doxazosin, glucocorticoids, betamethasone, dexamethasone, prednisolone, corticosterone, budesonide, sulfasalazine, rosiglitazone, mycophenolic acid, mesalamine, paclitaxel, 5-fluorouracil, cisplatin, vinblastine, vincristine, epothilones, methotrexate, azathioprine, adriamycin, mutamycin, endostatin, angiostatin, thymidine kinase inhibitors, cladribine, lidocaine, bupivacaine, ropivacaine, D-Phe-Pro-Arg chloromethyl ketone, platelet receptor antagonists, anti-thrombin antibodies, anti-platelet receptor antibodies, aspirin, dipyridamole, protamine, hirudin, prostaglandin inhibitors, platelet inhibitors, trapidil, liprostin, tick antiplatelet peptides, 5-azacytidine, vascular endothelial growth factors, growth factor receptors, transcriptional activators, translational promoters, antiproliferative agents, growth factor inhibitors, growth factor receptor antagonists, transcriptional repressers, translational repressors, replication inhibitors, inhibitory antibodies, antibodies directed against growth factors, bifunctional molecules consisting of a growth factor and a cytotoxin, bifunctional molecules consisting of an antibody and a cytotoxin, cholesterol lowering agents, vasodilating agents, agents which interfere with endogenous vasoactive mechanisms, radiochemical, antioxidants, probucol, antibiotic agents, penicillin, cefoxitin, oxacillin, tobranycin, anti-angiogenic agents, fibroblast growth factors, estrogen, estradiol (E2), estriol (E3), 17-beta estradiol, digoxin, beta blockers, captopril, enalopril, statins, steroids, vitamins, paclitaxel (as well as its derivatives, analogs or paclitaxel bound to proteins, *e.g.* Abraxanc™) 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, 2'-O-ester with N-(dimethylaminoethyl) glutamide hydrochloride salt, nitroglycerin, nitrous oxides, nitric oxides, antibiotics, aspirins, digitalis, estrogen, estradiol and glycosides. In one embodiment, the therapeutic agent is a smooth muscle cell inhibitor or antibiotic. In a preferred embodiment, the therapeutic agent is taxol (*e.g.*, Taxol®), or its analogs or derivatives. In another preferred embodiment, the therapeutic agent is paclitaxel, or its analogs or derivatives. In yet another preferred embodiment, the therapeutic agent is an antibiotic such as erythromycin, amphotericin, rapamycin, adriamycin, etc.

The term "genetic materials" means DNA or RNA, including, without limitation, of DNA/RNA encoding a useful protein stated below, intended to be inserted into a human body including viral vectors and non-viral vectors.

The term "biological materials" include cells, yeasts, bacteria, proteins, peptides, cytokines and hormones. Examples for peptides and proteins include vascular endothelial growth factor (VEGF), transforming growth factor (TGF), fibroblast growth factor (FGF), epidermal growth factor (EGF), cartilage growth factor (CGF), nerve growth factor (NGF), keratinocyte growth factor (KGF), skeletal growth factor (SGF), osteoblast-derived growth factor (BDGF), hepatocyte growth factor (HGF), insulin-like growth factor (IGF), cytokine growth factors (CGF), platelet-derived growth factor (PDGF), hypoxia inducible factor-1 (HIF-1), stem cell derived factor (SDF), stem cell factor (SCF), endothelial cell growth supplement (ECGS), granulocyte macrophage colony stimulating factor (OM-CSF), growth differentiation factor (GDF), integrin modulating factor (IMF), calmodulin (CaM), thymidine kinase (TK), tumor necrosis factor (TNF), growth hormone (GH), bone morphogenic protein (BMP) (*e.g.*, BMP-2, BMP-3, BMP-4, BMP-5, BMP-6 (Vgr-1), BMP-7 (PO-1), BMP-8, BMP-9, BMP-10, BMP-11, BMP-12, BMP-14, BMP-15, BMP-16, etc.), matrix metalloproteinase (MMP), tissue inhibitor of matrix metalloproteinase (TEMP), cytokines, interleukin (*e*.*g*., IL-1, IL-2, IL-3, IL-4, IL-5, IL-6, IL-7, IL-8, IL-9, IL-10, IL-11, IL-12, IL-15, etc.), lymphokines, interferon, integrin, collagen (all types), elastin, fibrillins, fibronectin, vitronectin, laminin, glycosaminoglycans, proteoglycans, transferrin, cytotactin, cell binding domains (*e*.*g*., ROD), and tenascin. Currently preferred BMP's are BMP-2, BMP-3, BMP-4, BMP-5, BMP-6, BMP-7. These dimeric proteins can be provided as homodimers, heterodimers, or combinations thereof, alone or together with other molecules. Cells can be of human origin (autologous or allogeneic) or from an animal source (xenogeneic), genetically engineered, if desired, to deliver proteins of interest at the transplant site. The delivery media can be formulated as needed to maintain cell function and viability. Cells include progenitor cells (*e.g*., endothelial progenitor cells), stem cells (*e.g.,* mesenchymal, hematopoietic, neuronal), stromal cells, parenchymal cells, undifferentiated cells, fibroblasts, macrophage, and satellite cells.

Other non-genetic therapeutic agents include:
- anti-thrombogenic agents such as heparin, heparin derivatives, urokinase, and PPack (dextrophenylalanine proline arginine chloromethylketone);
- anti-proliferative agents such as enoxaprin, angiopeptin, or monoclonal antibodies capable of blocking smooth muscle cell proliferation, hirudin, acetylsalicylic acid, tacrolimus, everolimus, amlodipine and doxazosin;
- anti-inflammatory agents such as glucocorticoids, betamethasone, dexamethasone, prednisolone, corticosterone, budesonide, estrogen, sulfasalazine, rosiglitazone, mycophenolic acid and mesalamine;
- anti-neoplastic/anti-proliferative/anti-miotic agents such as paclitaxel, 5-fluorouracil, cisplatin, vinblastine, vincristine, epothilones, methotrexate, azathioprine, adriamycin and mutamycin; endostatin, angiostatin and thymidine kinase inhibitors, cladribine, taxol and its analogs or derivatives;
- anesthetic agents such as lidocaine, bupivacaine, and ropivacaine;
- anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, aspirin (aspirin is also classified as an analgesic, antipyretic and anti-inflammatory drug), dipyridamole, protamine, hirudin, prostaglandin inhibitors, platelet inhibitors, antiplatelet agents such as trapidil or liprostin and tick antiplatelet peptides;
- DNA demethylating drugs such as 5-azacytidine, which is also categorized as a RNA or DNA metabolite that inhibit cell growth and induce apoptosis in certain cancer cells;
- vascular cell growth promoters such as growth factors, vascular endothelial growth factors (VEGF, all types including VEGF-2), growth factor receptors, transcriptional activators, and translational promoters;
- vascular cell growth inhibitors such as anti-proliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin;
- cholesterol-lowering agents, vasodilating agents, and agents which interfere with endogenous vasoactive mechanisms;
- anti-oxidants, such as probucol;
- antibiotic agents, such as penicillin, cefoxitin, oxacillin, tobranycin, rapamycin (sirolimus);
- angiogenic substances, such as acidic and basic fibroblast growth factors, estrogen including estradiol (E2), estriol (E3) and 17-beta estradiol;
- drugs for heart failure, such as digoxin, beta-blockers, angiotensin-converting enzyme (ACE) inhibitors including captopril and enalopril, statins and related compounds; and
- macrolide agents such as sirolimus, pimecrolimus, or tacrolimus, zotarolimus, everolimus.

Preferred biological materials include anti-proliferative drugs such as steroids, vitamins, and restenosis-inhibiting agents. Preferred restenosis-inhibiting agents include microtubule stabilizing agents such as Taxol®, paclitaxel (*i*.*e*., paclitaxel, paclitaxel analogs, or paclitaxel derivatives, and mixtures thereof). For example, derivatives suitable for use in the present invention include 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, and 2'-O-ester with N-(dimethylaminoethyl) glutamide hydrochloride salt

Other suitable therapeutic agents include tacrolimus; halofuginone; inhibitors of HSP90 heat shock proteins such as geldanamycin; microtubule stabilizing agents such as epothilone D; phosphodiesterase inhibitors such as cliostazole; Barket inhibitors; phospholamban inhibitors; and Serca 2 gene/proteins.

Other preferred therapeutic agents include nitroglycerin, nitrous oxides, nitric oxides, aspirins, digitalis, estrogen derivatives such as estradiol and glycosides.

In one embodiment, the therapeutic agent is capable of altering the cellular metabolism or inhibiting a cell activity, such as protein synthesis, DNA synthesis, spindle fiber formation, cellular proliferation, cell migration, microtubule formation, microfilament formation, extracellular matrix synthesis, extracellular matrix secretion, or increase in cell volume. In another embodiment, the therapeutic agent is capable of inhibiting cell proliferation and/or migration.

In certain embodiments, the therapeutic agents for use in the medical devices of the present invention can be synthesized by methods well known to one skilled in the art. Alternatively, the therapeutic agents can be purchased from chemical and pharmaceutical companies.

Methods suitable for applying therapeutic agents to the devices of the present invention preferably do not alter or adversely impact the therapeutic properties of the therapeutic agent.

### C. Suitable Polymers

Polymers useful for forming the coating compositions should be ones that are biocompatible, particularly during insertion or implantation of the device into the body and avoids irritation to body tissue. Examples of such polymers include, but not limited to, polyurethanes, polyisobutylene and its copolymers, silicones, and polyesters. Other suitable polymers include polyolefins, polyisobutylene, ethylene-alphaolefin copolymers, acrylic polymers and copolymers, vinyl halide polymers and copolymers such as polyvinyl chloride, polyvinyl ethers such as polyvinyl methyl ether, polyvinylidene halides such as polyvinylidene fluoride and polyvinylidene chloride, polyacrylonitrile, polyvinyl ketones, polyvinyl aromatics such as polystyrene, polyvinyl esters such as polyvinyl acetate; copolymers of vinyl monomers, copolymers of vinyl monomers and olefins such as ethylene-methyl methacrylate copolymers, acrylonitrile-styrene copolymers, ABS resins, ethylene-vinyl acetate copolymers, polyamides such as Nylon 66 and polycaprolactone, alkyd resins, polycarbonates, polyoxyethylenes, polyimides, polyethers, epoxy resins, polyurethanes, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chitins, polylactic acid, polyglycolic acid, and polylactic acid-polyethylene oxide copolymers. Since the polymer is being applied to a part of the medical device which undergoes mechanical challenges, *e*.*g*. expansion and contraction, the polymers are preferably selected from elastomeric polymers such as silicones (*e*.*g*. polysiloxanes and substituted polysiloxanes), polyurethanes, thermoplastic elastomers, ethylene vinyl acetate copolymers, polyolefin elastomers, and EPDM rubbers. The polymer is selected to allow the coating to better adhere to the surface of the strut when the stent is subjected to forces or stress. Furthermore, although the coating can be formed by using a single type of polymer, various combinations of polymers can be employed.

Generally, when a hydrophilic therapeutic agent is used then a hydrophilic polymer having a greater affinity for the therapeutic agent than another material that is less hydrophilic is preferred. When a hydrophobictherapeutic agent is used then a hydrophobic polymer having a greater affinity for the therapeutic agent is preferred.

Examples of suitable hydrophobic polymers or monomers include, but not limited to, polyolefins, such as polyethylene, polypropylene, poly(1-butene), poly(2-butene), poly(1-pentene), poly(2-pentene), poly(3-methyl-1-pentene), poly(4-methyl-1-pentene), poly(isoprene), poly(4-methyl-1-pentene), ethylene-propylene copolymers, ethylene-propylene-hexadiene copolymers, ethylene-vinyl acetate copolymers, blends of two or more polyolefins and random and block copolymers prepared from two or more different unsaturated monomers; styrene polymers, such as poly(styrene), poly(2-methylstyrene), styrene-acrylonitrile copolymers having less than about 20 mole-percent acrylonitrile, and styrene-2,2,3,3,-tetrafluoropropyl methacrylate copolymers; halogenated hydrocarbon polymers, such as poly(chlorotrifluoroethylene), chlorotrifluoroethylene-tetrafluoroethylene copolymers, poly(hexafluompmpylene), poly(tetrafluoroethylene), tetrafluoroethylene, tetrafluoroethylene-ethylene copolymers, poly(trifluoroethylene), poly(vinyl fluoride), and poly(vinylidene fluoride); vinyl polymers, such as poly(vinyl butyrate), poly(vinyl decanoate), poly(vinyl dodecanoate), poly(vinyl hexadecanoate), poly(vinyl hexanoate), poly(vinyl propionate), poly(vinyl octanoate), poly(heptafluoroisopropoxyethylene), poly(heptafluoroisopropoxypropylene), and poly(methacrylonitrile); acrylic polymers, such as poly(n-butyl acetate), poly(ethyl acrylate), poly(1-chorodifluoromethyl)tetrafluoroethyl acrylate, poly di(chlorofluoromethyl)fluoromethyl acrylate, poly(1,1-dihydroheptafluorobutyl acrylate), poly(1,1-dihydropentafluoroisopropyl acrylate), poly(1,1-dihydropentadecafluorooctyl acrylate), poly(heptafluoroisopropyl acrylate), poly 5-(heptafluoroisopropoxy)pentyl acrylate, poly 11-(heptafluoroisopropoxy)undecyl acrylate, poly 2-(heptafluoropropoxy)ethyl acrylate, and poly(nonafluoroisobutyl acrylate); methacrylic polymers, such as poly(benzyl methacrylate), poly(n-butyl methacrylate), poly(isobutyl memacrylate), poly(t-butyl methacrylate), poly(t-butylaminoethyl methacrylate), poly(dodecyl methacrylate), poly(ethyl methacrylate), poly(2-ethylhexyl methacrylate), poly(n-hexyl methacrylate), poly(phenyl methacrylate), poly(n-propyl methacrylate), poly(octadecyl methacrylate), poly(1,1-dihydropentadecafluorooctyl methacrylate), poly(heptafluoroisopropyl methacrylate), poly(heptadecafluorooctyl methacrylate), poly(1-hydrotetrafluoroethyl methacrylate), poly(1,1-dihydrotetrafluoropropyl methacrylate), poly(1-hydrohexafluoroisopropyl methacrylate), and poly(t-nonafluorobutyl methacrylate); polyesters, such a poly(ethylene terephthalate) and poly(butylene terephthalate); condensation type polymers such as and polyurethanes and siloxane-urethane copolymers; polyorganosiloxanes, *i*.*e*., polymeric materials characterized by repeating siloxane groups, represented by Ra SiO 4-a/2, where R is a monovalent substituted or unsubstituted hydrocarbon radical and the value of a is 1 or 2; and naturally occurring hydrophobic polymers such as rubber.

Examples of suitable hydrophilic polymers or monomers include, but not limited to; (meth)acrylic acid, or alkaline metal or ammonium salts thereof; (meth)acrylamide; (meth)acrylonitrile; those polymers to which unsaturated dibasic, such as maleic acid and fumaric acid or half esters of these unsaturated dibasic acids, or alkaline metal or ammonium salts of these dibasic adds or half esters, is added; those polymers to which unsaturated sulfonic, such as 2-acrylamido-2-methylpropanesulfonic, 2-(meth)acryloylethanesulfonic acid, or alkaline metal or ammonium salts thereof, is added; and 2-hydroxyethyl (meth)acrylate and 2-hydroxypropyl (meth)acrylate.

Polyvinyl alcohol is also an example of hydrophilic polymer. Polyvinyl alcohol may contain a plurality of hydrophilic groups such as hydroxyl, amido, carboxyl, amino, ammonium or sulfonyl (-SO3). Hydrophilic polymers also include, but are not limited to, starch, polysaccharides and related cellulosic polymers; polyalkylene glycols and oxides such as the polyethylene oxides; polymerized ethylenically unsaturated carboxylic acids such as acrylic, methacrylic and maleic acids and partial esters derived from these acids and polyhydric alcohols such as the alkylene glycols; homopolymers and copolymers derived from acrylamide; and homopolymers and copolymers of vinylpyrrolidone.

Preferably, for stents which undergo mechanical challenges, e.g., expansion and contraction, polymers should be selected from elastomeric polymers such as silicones (e.g., polysiloxanes and substituted polysiloxanes), polyurethanes, thermoplastic elastomers, ethylene vinyl acetate copolymers, polyolefin elastomers, and EPDM rubbers. Because of the elastic nature of these polymers, the coating composition is capable of undergoing deformation under the yield point when the device is subjected to forces, stress or mechanical challenge.

### D. Methods for Making the Coatings

The coating compositions suitable for the device described herein can be prepared by dissolving or suspending a polymer and/or therapeutic agent in a solvent. Solvents that may be used to prepare coating compositions include ones which can dissolve or suspend the polymer and/or therapeutic agent in solution. Examples of suitable solvents include, but are not limited to, water, such as hot water, tetrahydrofuran, methylethylketone, chloroform, toluene, acetone, isooctane, 1,1,1, trichloroethane, dichloromethane, isopropanol, IPA, and mixture thereof.

The aforementioned coated medical devices can be made by applying coating compositions onto the surface of the medical device. Coating compositions can be applied by any method to a surface of a medical device or to another coating composition known by one skilled in the art. The different surfaces may be coated by the same or different methods. Suitable methods for applying the coating compositions to the medical devices include, but are not limited to, spray-coating, painting, rolling, electrostatic deposition, ink jet coating, and a batch process such as air suspension, pan-coating or ultrasonic mist spraying, or a combination thereof.

In embodiments where a coating composition is to be applied to fewer than all the surfaces of the struts of a stent, such as on the stents described above in **Figure 4a** it is preferable to employ coating methods that selectively apply the coating composition. For instance, a first or second coating composition can be deposited onto a substrate. The substrate is preferably made from materials that has minimal adhesion to the coating composition so that the coating composition can be easily removed and transferred to the surface. For instance, in the embodiment of **Figure 4a****,** the first coating composition **22** can be applied to the abluminal surface **14,** luminal surface **16** and side surfaces **18.** The second coating composition **24** can be applied to the luminal surface **16** and side surface **18** of the struts by contacting those surfaces with the substrate having the second coating composition **24** coated thereon to transfer the second coating composition **24** to the luminal surface **16** and side surface **18** of the struts.

Also, it may be preferable to mask or cover the surface that is not to be coated with a particular coating composition. For instance, to avoid having the first coating composition disposed upon the luminal surface 16 and side surfaces **18** of the strut, the luminal surface **16** and side surfaces **18** can be masked.

The luminal surface **16** and side surfaces **18** can be masked, for instance, by application of a protective wrap to that surface. The protective wrap is a material that would protect the coated surface from exposure to the coating applied to the opposing surface. Suitable material for this protective wrap include, for example, PTFE film, dyna-leap, Kapton®, or any other appropriate type of covering or wrapping material. The protective wrap serves to protect the luminal surface **16** and side surfaces **18** from exposure to the coating composition as it is being applied to the abluminal surface **14.** Thus, the protective wrap will protect a luminal surface **16** and side surfaces **18** that has been already coated from additional deposition of the coating to be applied to the abluminal surface **14.** After the abluminal surface **14** of the struts **12** of the medical device have been coated, the wrap covering the luminal surface **16** and side surfaces **18** may be removed.

In one embodiment where only the luminal surface **16** is to be masked, the luminal surface **16** can be masked by placing the stent on a mandrel. The luminal surface **16** which is placed against the mandrel will not be exposed to a coating composition that is applied to the abluminal surface **14.** For example, in an embodiment, the stent that is mounted on the mandrel may then be rolled over a substrate containing a coating composition to transfer the coating composition to the abluminal surface **14** and side surfaces **18.** Alternatively, the stent can be placed on the mandrel and the abluminal surface 14 and side surfaces **18** of the strut are spray-coated with the coating composition. In yet another embodiment, it is possible to coat all surfaces of the stents, and remove the coating from desired areas by methods such as laser ablation.

For spray coating, a nozzle assembly may be used to spray a coating composition onto the inner surface. The nozzle assembly may be in the form of a cone that sprays the coating composition at an angle. The angle of the spray from the nozzles may need to be adjusted to ensure uniform thickness of the coating on the inner surface. Also, a nozzle assembly with small spray nozzles can be inserted into one end of the stent and moved through the stent until it extends past the opposite end of the stent. Preferably, the spray mist flow is started while the nozzle is still outside of the stent. This step places a coating composition on the inside surface and one side surface of the struts of the stent. The coating process may be repeated again. Preferably, the spray nozzle is inserted into the other end of the stent to coat the other side surface of the struts. By repeating the spraying from two directions, both side surfaces are coated with a coating composition.

According to the invention, there is more than one coating composition applied to the stent. In one embodiment, the second coating composition is free of the therapeutic agent when applied to the first coating composition. In other embodiments, the second coating composition is free of any therapeutic agent. In still other embodiments, the second coating composition can comprise a therapeutic agent. In some embodiments, the first coating composition is in the form of a first coating layer and the second coating composition is in the form of a second coating layer.

During the process of coating, the coating thickness can be controlled. In certain embodiments, the first coating layer has a thickness of about - 1 micrometer to about 20 micrometer. In other embodiments, the second coating layer has a thickness of about - 0.5 micrometer to about - 20 micrometer. In particular embodiments, the second coating layer has a thickness of less then 5 µm. In preferred embodiments, the first coating composition and second coating composition are coated to conform to the surfaces of the strut to preserve the openings of the sidewall structure.

After a coating composition has been applied, it can be cured. Curing is defined as the process of converting the polymeric material into the finished or useful state by the application of heat, vacuum, and/or chemical agents which induce physico-chemical changes. The applicable time and temperature for curing are determined by the particular polymer involved and particular therapeutic agent used, if any, as known by one skilled in the act. The coated medical devices may thereafter be subjected to a post-cure process wherein the medical devices are exposed to a low energy for stabilization of the coating. Also, after the medical device is coated, it preferably should be sterilized by methods of sterilization as known in the art.

In use, a coated medical device, such as an expandable stent, according to the present invention can be made to provide desired release profile of the therapeutic agent. The medical devices and stents of the present invention may be used for any appropriate medical procedure. Delivery of the medical device can be accomplished using methods well known to those skilled in the art, such as mounting the stent on an inflatable balloon disposed at the distal end of a delivery catheter.

## Claims

1. A stent designed for implantation into a blood vessel of a patient comprising:
a tubular stent sidewall structure comprising a plurality of struts (12) and openings in the sidewall structure; wherein at least one strut (12) comprises an abluminal surface (14), and a luminal surface (16) opposite the abluminal surface (14);
a first coating composition (22) comprising a first polymer and a therapeutic agent disposed on the abluminal surface (14) and luminal surface (16); and
a second coating composition (24) disposed on a portion of the first coating composition (22) that is disposed on the luminal surface (16) wherein the second coating composition (24) is biodegradable and comprises a bioabsorbable polymer; **characterised in that**
the abluminal surface (14) is free of the second coating composition (24); and **in that** when the stent is implanted, the first coating composition (22) disposed on the abluminal surface (14) is in direct contact with the blood vessel.

2. The stent of claim 1, wherein the strut further comprises a first side surface (18) and a second side surface (18) opposite the first side surface (18), in which each side surface (18) is disposed between the abluminal and luminal surfaces (14, 16).

3. The stent of claim 2, wherein the first coating composition (22) is disposed on the first and second side surfaces (18) of the strut (12); and the second coating composition (24) is disposed on a portion of the first coating composition (22) that is disposed on each of the first and second side surfaces (18).

4. The stent of claim 1, wherein the first polymer is biostable.

5. The stent of claim 1, wherein the therapeutic agent comprises an anti-thrombogenic agent, anti-angiogensis agent, anti-proliferative agent, anti-restenosis agent, growth factor, antibiotic or radiochemical.

6. The stent of claim 1, wherein the therapeutic agent comprises an agent that inhibits smooth muscle cell proliferation.

7. The stent of claim 1, wherein the therapeutic agent comprises paclitaxel, sirolimus, everolimus, pimecrolimus or tacrolimus.

8. The stent of claim 1, wherein the second coating composition (24) is free of the therapeutic agent when applied to the first coating composition (22).

9. The stent of claim 1, wherein the bioabsorbable polymer comprises a polyelectrolyte component

10. The stent of claim 1, wherein the first coating composition (22) and second coating composition (24) conform to the surfaces of the strut (12) to preserve the openings of the side wall structure.

11. The stent of claim 1, wherein the stent is an intravascular stent.

12. The stent of claim 11 wherein at least one strut (12) comprises a first side surface (18) and a second side surface (18) opposite the first side surface (18), in which each side surface (18) is disposed between the abluminal and luminal surfaces (14, 16);
and wherein the first polymer is a biostable polymer and the first coating composition (22) comprises an anti-restenosis agent and is disposed on the abluminal surface (14), the luminal surface (16) and side surfaces (18);
and wherein the second coating composition (24) is disposed on the first side surface (18) and second side surface (18), wherein the abluminal surface (14) is free of the second coating composition (24);
and wherein when the stent is implanted, the first coating composition (22) disposed on the abluminal surface (14) is in direct contact with the blood vessel.

## Patentansprüche

1. Stent, ausgestaltet für die Implantation in ein Blutgefäß eines Patienten, umfassend:
eine röhrenförmige Stent-Seitenwandstruktur, umfassend eine Mehrzahl Streben (12) und Öffnungen in der Seitenwandstruktur; wobei mindestens eine Strebe (12) eine abluminale Fläche (14) und eine luminale Fläche (16) gegenüberliegend der abluminalen Fläche (14) aufweist;
eine erste Beschichtungszusammensetzung (22), umfassend ein erstes Polymer und ein Behandlungsmittel, die auf der abluminalen Fläche (14) und der luminalen Fläche (16) angeordnet sind; und
eine zweite Beschichtungszusammensetzung (24), die auf einem Abschnitt der ersten Beschichtungszusammensetzung (22) angeordnet ist, die auf der luminalen Fläche (16) angeordnet ist, wobei die zweite Beschichtungszusammensetzung (24) biologisch abbaubar ist und ein bioresorbierbares Polymer umfasst; **dadurch gekennzeichnet, dass**
die abluminale Fläche (14) frei von der zweiten Beschichtungszusammensetzung (24) ist; und dass, wenn der Stent implantiert ist, die erste Beschichtungszusammensetzung (22), die auf der abluminalen Fläche (14) angeordnet ist, in direktem Kontakt mit dem Blutgefäß ist.

2. Stent nach Anspruch 1, wobei die Strebe überdies eine erste Seitenfläche (18) und eine zweite Seitenfläche (18) gegenüberliegend der ersten Seitenfläche (18) umfasst, wobei jede Seitenfläche (18) zwischen der abluminalen und luminalen Fläche (14, 16) angeordnet ist.

3. Stent nach Anspruch 2, wobei die erste Beschichtungszusammensetzung (22) auf der ersten und zweiten Seitenfläche (18) der Strebe (12) angeordnet ist; und die zweite Beschichtungszusammensetzung (24) auf einem Abschnitt der ersten Beschichtungszusammensetzung (22) angeordnet ist, die auf jeder, der ersten und zweiten Seitenfläche (18), angeordnet ist.

4. Stent nach Anspruch 1, wobei das erste Polymer biostabil ist.

5. Stent nach Anspruch 1, wobei das Behandlungsmittel ein antithrombogenes Mittel, ein antiangiogenes Mittel, ein antiproliferatives Mittel, ein Antirestenose-Mittel, Wachstumsfaktor, antibiotisch oder radiochemisch umfasst.

6. Stent nach Anspruch 1, wobei das Behandlungsmittel ein Mittel umfasst, das die Wucherung von weichen Muskelzellen hemmt.

7. Stent nach Anspruch 1, wobei das Behandlungsmittel Paclitaxel, Sirolimus, Everolimus, Pimecrolimus oder Tacrolimus umfasst.

8. Stent nach Anspruch 1, wobei die zweite Beschichtungszusammensetzung (24) frei von dem Behandlungsmittel ist, wenn sie auf die erste Beschichtungszusammensetzung (22) aufgebracht wird.

9. Stent nach Anspruch 1, wobei das bioresorbierbare Polymer eine Polyelektrolytkomponente umfasst.

10. Stent nach Anspruch 1, wobei die erste Beschichtungszusammensetzung (22) und die zweite Beschichtungszusammensetzung (24) mit den Flächen der Strebe (12) übereinstimmen, um die Öffnungen der Seitenwandstruktur zu erhalten.

11. Stent nach Anspruch 1, wobei der Stent ein intravaskulärer Stent ist.

12. Stent nach Anspruch 11, wobei mindestens eine Strebe (12) eine erste Seitenfläche (18) und eine zweite Seitenfläche (18) gegenüberliegend der ersten Seitenfläche (18) umfasst, wobei jede Seitenfläche (18) zwischen der abluminalen und luminalen Fläche (14, 16) angeordnet ist;
und wobei das erste Polymer ein biostabiles Polymer ist und die erste Beschichtungszusammensetzung (22) ein Antirestenose-Mittel umfasst und auf der abluminalen Fläche (14), der luminalen Fläche (16) und den Seitenflächen (18) angeordnet ist;
und wobei die zweite Beschichtungszusammensetzung (24) auf der ersten Seitenfläche (18) und der zweiten Seitenfläche (18) angeordnet ist, wobei die abluminale Fläche (14) frei von der zweiten Beschichtungszusammensetzung (24) ist;
und wobei, wenn der Stent implantiert ist, die erste Beschichtungszusammensetzung (22), die auf der abluminalen Fläche (14) angeordnet ist, in direktem Kontakt mit dem Blutgefäß ist.

## Revendications

1. Endoprothèse vasculaire conçue pour l'implantation dans un vaisseau sanguin d'un patient comprenant :
une structure tubulaire de paroi latérale d'endoprothèse vasculaire comprenant une pluralité d'entretoises (12) et d'ouvertures dans la structure de paroi latérale ; dans laquelle au moins une entretoise (12) comprend une surface abluminale (14), et une surface luminale (16) opposée à la surface abluminale (14) ;
une première composition de revêtement (22) comprenant un premier polymère et un agent thérapeutique disposé sur la surface abluminale (14) et la surface luminale (16) ; et
une seconde composition de revêtement (24) disposée sur une portion de la première composition de revêtement (22) qui est disposée sur la surface luminale (16) dans laquelle la seconde composition de revêtement (24) est biodégradable et comprend un polymère bioabsorbable ; **caractérisée en ce que**
la surface abluminale (14) est dénuée de la seconde composition de revêtement (24) ; et **en ce que** quand l'endoprothèse vasculaire est implantée, la première composition de revêtement (22) disposée sur la surface abluminale (14) est en contact direct avec le vaisseau sanguin.

2. Endoprothèse vasculaire selon la revendication 1, dans laquelle l'entretoise comprend en outre une première surface latérale (18) et une seconde surface latérale (18) opposée à la première surface latérale (18), dans laquelle chaque surface latérale (18) est disposée entre les surfaces abluminale et luminale (14, 16).

3. Endoprothèse vasculaire selon la revendication 2, dans laquelle la première composition de revêtement (22) est disposée sur les première et seconde surfaces latérales (18) de l'entretoise (12) ; et la seconde composition de revêtement (24) est disposée sur une portion de la première composition de revêtement (22) qui est disposée sur chacune des première et seconde surfaces latérales (18).

4. Endoprothèse vasculaire selon la revendication 1, dans laquelle le premier polymère est biostable.

5. Endoprothèse vasculaire selon la revendication 1, dans laquelle l'agent thérapeutique comprend un agent anti-thrombogène, agent anti-angiogénique, agent anti-prolifératif, agent anti-resténose, facteur de croissance, antibiotique ou radiochimique.

6. Endoprothèse vasculaire selon la revendication 1, dans laquelle l'agent thérapeutique comprend un agent qui empêche la prolifération de cellules musculaires lisses.

7. Endoprothèse vasculaire selon la revendication 1, dans laquelle l'agent thérapeutique comprend du paclitaxel, du sirolimus, de l'évérolimus, du pimécrolimus ou du tacrolimus.

8. Endoprothèse vasculaire selon la revendication 1, dans laquelle la seconde composition de revêtement (24) est dénuée de l'agent thérapeutique quand appliquée à la première composition de revêtement (22).

9. Endoprothèse vasculaire selon la revendication 1, dans laquelle le polymère bioabsorbable comprend un composant polyélectrolyte.

10. Endoprothèse vasculaire selon la revendication 1, dans laquelle la première composition de revêtement (22) et la seconde composition de revêtement (24) se conforment aux surfaces de l'entretoise (12) pour préserver les ouvertures de la structure de paroi latérale.

11. Endoprothèse vasculaire selon la revendication 1, dans laquelle l'endoprothèse vasculaire est une endoprothèse intravasculaire.

12. Endoprothèse vasculaire selon la revendication 11, dans laquelle au moins une entretoise (12) comprend une première surface latérale (18) et une seconde surface latérale (18) opposée à la première surface latérale (18), dans laquelle chaque surface latérale (18) est disposée entre les surfaces abluminale et luminale (14, 16) ;
et dans laquelle le premier polymère est un polymère biostable et la première composition de revêtement (22) comprend un agent anti-resténose et est disposée sur la surface abluminale (14), la surface luminale (16) et les surfaces latérales (18) ;
et dans laquelle la seconde composition de revêtement (24) est disposée sur la première surface latérale (18) et la seconde surface latérale (18), dans laquelle la surface abluminale (14) est dénuée de la seconde composition de revêtement (24) ;
et dans laquelle quand l'endoprothèse vasculaire est implantée, la première composition de revêtement (22) disposée sur la surface abluminale (14) est en contact direct avec le vaisseau sanguin.
